# EUROPEAN PATENT APPLICATION

(11) **EP 4 778 442 A1**
(43) Date of publication of application: **22.07.2026**
(21) Application number: 26150806.3
(22) Date of filing: 08.01.2026
(51) Int. Cl.: A61B 1/00, G02B 23/24, A61B 1/002

(54) **SELF-ADAPTIVE ENDOSCOPE WORKING DISTANCE ADJUSTMENT SYSTEM AND ENDOSCOPIC IMAGING SYSTEM**

(30) Priority: 21.01.2025 CN 202510089584
(71) Applicant: Anhui Dendrite Optical Technology Co., Ltd., Hefei City, Anhui Province 230000 (CN)
(72) Inventor: LIAO, Jiasheng, Hefei City, Anhui Province, 230000 (CN); DOU, Huainan, Hefei City, Anhui Province, 230000 (CN); WAN, Liping, Hefei City, Anhui Province, 230000 (CN); YAN, Ping, Hefei City, Anhui Province, 230000 (CN); DENG, Yin, Hefei City, Anhui Province, 230000 (CN); CHIANG, LI-Yang, Hefei City, Anhui Province, 230000 (CN); WENG, Xianjie, Hefei City, Anhui Province, 230000 (CN)
(74) Representative: Gille Hrabal Partnerschaftsgesellschaft mbB Patentanwälte

(57) **Abstract**

The present invention provides a self-adaptive endoscope working distance adjustment system and an endoscopic imaging system. The self-adaptive endoscope working distance adjustment system includes: an image relay sheath, a lens holder, a protective sheath, and a working distance auxiliary adjustment system disposed between the protective sheath and the lens holder and/or between the protective sheath and the image relay sheath. The lens holder axially protrudes to form a support portion, and an imaging lens is disposed inside the image relay sheath away from an image side. The protective sheath includes: an outer sheath tube axially extending over the imaging lens, a sheath tube connection portion partially sleeved on an outer side of the support portion and connected to the outer sheath tube, and a protective lens disposed at an object-side end inside the outer sheath tube. The working distance auxiliary adjustment system is configured to adjust a relative axial position between the imaging lens and the protective lens and to maintain a working distance between the protective lens and the imaging lens. This application achieves an optimal object distance of the endoscopic imaging system by using the working distance auxiliary adjustment system, ensuring the quality of microscopic imaging.

## Description

### FIELD OF TECHNOLOGY

The present invention relates to the field of endomicroscopic technology, and in particular to a self-adaptive endoscope working distance adjustment system and an endoscopic imaging system.

### BACKGROUND

Endomicroscopes are widely used in the field of medical diagnosis and treatment for observing and handling microscopic tissue structures. The endomicroscopes provide clinicians with cellular-level imaging support in clinical practice, offering a reliable basis for accurate lesion assessment. Because the endomicroscopes are in direct contact with human tissue and provide cellular-level images, with magnifications usually exceeding 1,000x, any error of 0.1 mm can significantly affect imaging quality. Therefore, it is crucial to precisely maintain the endomicroscope at an optimal working distance (namely, an optimal imaging distance from an object lens to the human tissue).

In the prior art, traditional endomicroscopes typically maintain the optimal working distance by using fastened protective lenses or other adjustment structures. However, the working distance of the endomicroscope may vary slightly due to an assembly tolerance, making it difficult to achieve an optimal object distance, thereby directly affecting the quality of microscopic imaging.

In view of the above, it is necessary to improve the endomicroscopes in the prior art to solve the above problems.

### SUMMARY

The present invention is intended to disclose a self-adaptive endoscope working distance adjustment system and an endoscopic imaging system, to resolve various defects existing in endomicroscopes of the prior art, especially to achieve an optimal object distance in the endoscopic imaging system and ensure the quality of microscopic imaging.

To achieve the above purpose, in a first aspect, the present invention provides a self-adaptive endoscope working distance adjustment system, including: an image relay sheath, a lens holder disposed on an image side of the image relay sheath, a protective sheath sleeved on an outer side of the image relay sheath, and a working distance auxiliary adjustment system disposed between the protective sheath and the lens holder and/or between the protective sheath and the image relay sheath, where
the lens holder axially protrudes to form a support portion, and an imaging lens is disposed inside the image relay sheath away from the image side;
the protective sheath includes: an outer sheath tube axially extending over the imaging lens, a sheath tube connection portion partially sleeved on an outer side of the support portion and connected to the outer sheath tube, and a protective lens disposed at an object-side end inside the outer sheath tube; and
the working distance auxiliary adjustment system is configured to adjust a relative axial position between the imaging lens and the protective lens and to maintain a working distance between the protective lens and the imaging lens.

As a further improvement of the present invention, the working distance auxiliary adjustment system is configured as a first retaining ring sleeved on the outer side of the support portion;
at least part of an outer periphery of the support portion forms a first guiding portion, and the sheath tube connection portion is connected to the support portion through the first guiding portion; and
when the sheath tube connection portion is rotated axially relative to the support portion, the first guiding portion guides the sheath tube connection portion to move axially, so that after an axial position of the protective lens relative to the imaging lens is adjusted, the first retaining ring is axially abutted between the sheath tube connection portion and the support portion.

As a further improvement of the present invention, the working distance auxiliary adjustment system is configured as a second retaining ring sleeved on the outer side of the image relay sheath;
at least part of an outer periphery of the support portion forms a first guiding portion, and the sheath tube connection portion is connected to the support portion through the first guiding portion; and
when the sheath tube connection portion is rotated axially relative to the support portion, the first guiding portion guides the sheath tube connection portion to move axially, so that after an axial position of the protective lens relative to the imaging lens is adjusted, the second retaining ring is axially abutted between the sheath tube connection portion and the support portion.

As a further improvement of the present invention, after the protective sheath is sleeved onto the support portion, a sealed space is formed between the protective sheath and the image relay sheath.

As a further improvement of the present invention, the first guiding portion is configured as an external thread portion, and an internal thread portion engaged with the external thread portion is formed on an inner periphery of the sheath tube connection portion.

As a further improvement of the present invention, the sheath tube connection portion includes: a holding portion for fastening the outer sheath tube, and an adjusting portion forming the internal thread portion for connecting to the support portion. As a further improvement of the present invention, the lens holder radially protrudes to form a protruding portion that axially abuts against the first retaining ring, and the adjusting portion axially abuts against a side of the first retaining ring opposite to the protruding portion.

As a further improvement of the present invention, the support portion axially abuts against the second retaining ring, and the holding portion axially abuts against a side of the second retaining ring opposite to the support portion.

In a second aspect, the present invention further provides an endoscopic imaging system, including: the self-adaptive endoscope working distance adjustment system described in the first aspect, a relay lens coaxially disposed within an image relay sheath included in the self-adaptive endoscope working distance adjustment system, a dichroic mirror disposed within a lens holder included in the self-adaptive endoscope working distance adjustment system, and a light source; and
the endoscopic imaging system is configured to perform optical imaging on biological tissue.

Compared with the prior art, the present invention has the following beneficial effects. During adjustment of the optimal object distance, the present invention adjusts an axial position of the protective lens relative to the imaging lens by using the working distance auxiliary adjustment system, thereby adjusting the axial distance between the protective lens and the imaging lens, so that the relative position between the protective lens and the imaging lens is maintained within the optimal range, ensuring that the imaging lens and the protective lens are at an optimal working distance, thus achieving an optimal object distance and ensuring high-quality microscopic imaging.

### BRIEF DESCRIPTION OF THE DRAWINGS

FIG. 1 is an overall schematic view of a self-adaptive endoscope working distance adjustment system disclosed in the present invention, where a working distance auxiliary adjustment system is configured as a first retaining ring sleeved on an outer side of a support portion;
FIG. 2 is a schematic view of the working distance auxiliary adjustment system configured as a second retaining ring sleeved on an outer side of an image relay sheath;
FIG. 3 is a schematic view of the working distance auxiliary adjustment system configured as a movable cylinder partially sleeved on an outer side of a sheath tube connection portion and rotatably connected to a lens holder;
FIG. 4 is a schematic view of the working distance auxiliary adjustment system configured as a spacer ring axially abutted between an imaging lens and a protective lens;
FIG. 5 is a sectional view along arrow A-A in FIG. 3; and
FIG. 6 is an overall schematic view of an endoscopic imaging system including the self-adaptive endoscope working distance adjustment system disclosed in the present invention.

### DESCRIPTION OF THE EMBODIMENTS

The following describes the present invention in detail with reference to implementations shown in the accompanying drawings, but it should be noted that these implementations do not impose limitations on the present invention. Functional, methodological, or structural equivalent transformations or substitutions made by persons of ordinary skill in the art according to these implementations all fall into the protection scope of the present invention.

Drawings in the present invention are not strictly drawn to actual scale, and specific dimensions of structures can be determined according to actual needs. The drawings described in the present invention are merely structural schematic diagrams.

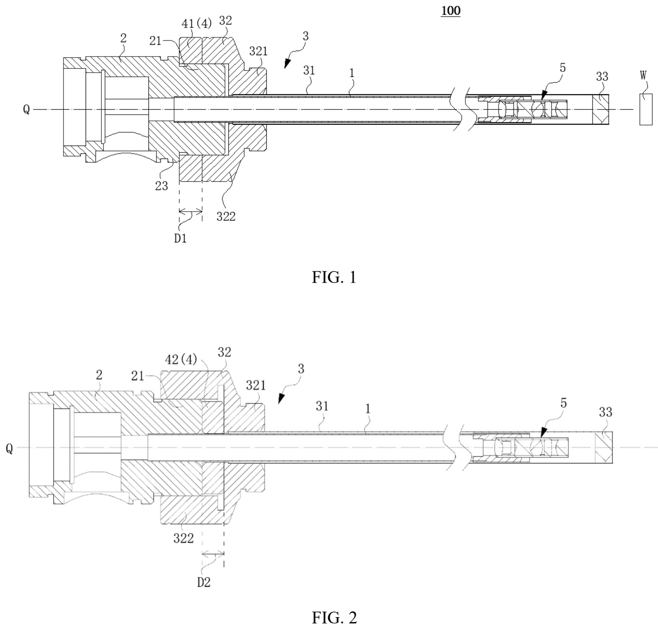

It should be noted that in the following embodiments, a term "optical axis" means an optical axis Q in FIG. 1. A term "axial" means a direction parallel to the optical axis Q. From the perspectives shown in FIG. 1 to FIG. 4, as shown in FIG. 1, a direction in which an object under inspection W is located is an object side, and a direction opposite to the object under inspection W is an image side. The object under inspection W includes living organisms or ex vivo biological tissues.

Refer to FIG. 1 to FIG. 6 for a specific implementation of a self-adaptive endoscope working distance adjustment system and an endoscopic imaging system disclosed.

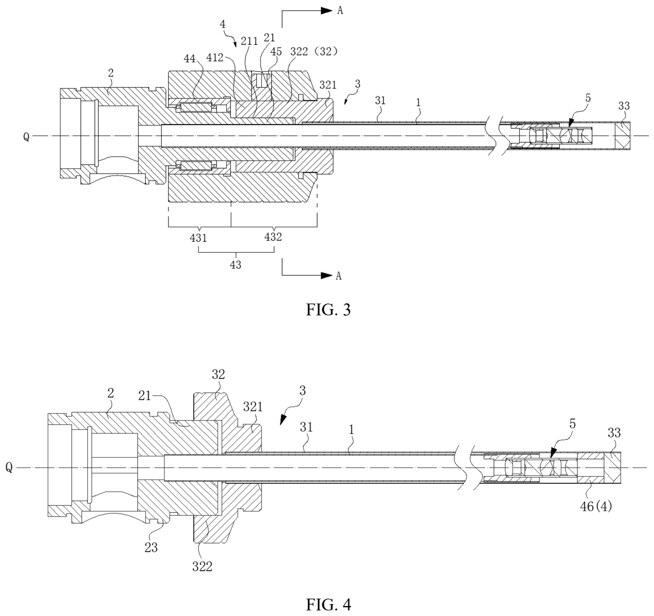

As shown in FIG. 1 and FIG. 4, the self-adaptive endoscope working distance adjustment system 100 can be installed on an optical inspection device used for observing biological bodies (including living organisms or ex vivo biological tissues), and a relative axial position between an imaging lens 5 and a protective lens 33 is adjusted through a working distance auxiliary adjustment system 4, to ensure an optimal working distance and the quality of microscopic imaging. Specifically, the optical inspection device may be, for example, an endoscope. The endoscope is a minimally invasive medical tool for a human body and is designed to enter the human body via a natural cavity of the human body or small surgical incision, and the endoscope is guided into biological tissue to be examined, to obtain corresponding images of the tissue to be examined by medical personnel. In addition, the applicant indicates that the endoscope is used as an exemplary illustration in the following description. Of course, the device may alternatively be a device used for flaw detection in an industrial field, which is not limited to this embodiment, but the scope of protection of this application shall not be limited.

As shown in FIG. 1 to FIG. 4, in this implementation, the self-adaptive endoscope working distance adjustment system 100 includes: an image relay sheath 1, a lens holder 2 disposed on an image side of the image relay sheath 1, a protective sheath 3 sleeved on an outer side of the image relay sheath 1, and a working distance auxiliary adjustment system 4 disposed between the protective sheath 3 and the lens holder 2 and/or between the protective sheath 3 and the image relay sheath 1. The lens holder 2 axially protrudes to form a support portion 21, and an imaging lens 5 is disposed inside the image relay sheath 1 away from the image side. The protective sheath 3 includes: an outer sheath tube 31 axially extending over the imaging lens 5, a sheath tube connection portion 32 partially sleeved on an outer side of the support portion 21 and connected to the outer sheath tube 31, and a protective lens 33 disposed at an object-side end inside the outer sheath tube 31. The working distance auxiliary adjustment system 4 is configured to adjust a relative axial position between the imaging lens 5 and the protective lens 33 and to maintain a working distance between the protective lens 33 and the imaging lens 5.

The self-adaptive endoscope working distance adjustment system 100 adjusts, during adjustment of an optimal object distance, an axial position of the protective lens 33 relative to the imaging lens 5 by using the working distance auxiliary adjustment system 4, thereby adjusting an axial distance between the protective lens 33 and the imaging lens 5, so that the relative position between the protective lens 33 and the imaging lens 5 is maintained within an optimal range, ensuring an optimal working distance between the imaging lens 5 and the protective lens 33, thus achieving an optimal object distance and ensuring high-quality microscopic imaging. After adjustment completion, an adjusted position (a position relative to the imaging lens 5) of the protective lens 33 is fastened by using the working distance auxiliary adjustment system 4, ensuring that the working distance between the protective lens 33 and the imaging lens 5 is maintained at a stable value after adjustment completion, preventing changes in the working distance due to an external factor (such as vibration or temperature changes), thereby maintaining the optimal object distance.

Compared with endomicroscopes in the prior art that are difficult to achieve the optimal object distance due to an assembly tolerance, the self-adaptive endoscope working distance adjustment system 100 maintains the relative position between the protective lens 33 and the imaging lens 5 within the optimal range based on an adjustment mechanism of the working distance auxiliary adjustment system 4, to achieve the optimal object distance between the imaging lens 5 and the protective lens 33, thereby solving a problem in the prior art of a difficulty in achieving the optimal object distance.

In some examples, as shown in FIG. 1, the image relay sheath 1 is configured to support and fasten the imaging lens 5, so that after the working distance between the protective lens 33 and the imaging lens 5 is adjusted, a position of the imaging lens 5 is stably maintained, preventing the imaging lens 5 from being displaced by an external factor during operation.

In some examples, as shown in FIG. 1, the lens holder 2 is located on the image side of the image relay sheath 1, and the image relay sheath 1 axially extends into the support portion 21, providing stable support for the image relay sheath 1 and the protective sheath 3 through the support portion 21, ensuring stable assembly of the image relay sheath 1 and the protective sheath 3, so that the image relay sheath 1 and the protective sheath 3 are always in stable relative positions during operation.

In some examples, as shown in FIG. 1, the protective sheath 3 is sleeved on the outer side of the image relay sheath 1 to protect the imaging lens 5 from external substances such as dust, moisture, and other potential contaminants. The outer sheath tube 31 extends axially, covering and protecting the imaging lens 5. The protective sheath 3 is connected to the support portion 21 through the sheath tube connection portion 32, ensuring the stability of the protective sheath 3. The protective lens 33 is located at the object-side end of the outer sheath tube 31, further protecting the imaging lens 5 and reducing optical interference, minimizing the impact of an external light source on the quality of microscopic imaging, and ensuring the clarity and quality of microscopic imaging.

In some examples, as shown in FIG. 1, the working distance auxiliary adjustment system 4 is configured to precisely adjust an axial position between the imaging lens 5 and the protective lens 33 and to make adjustments according to actual assembly tolerances, ensuring the optimal working distance between the imaging lens 5 and the protective lens 33. After adjustment completion, the working distance between the imaging lens 5 and the protective lens 33 is measured by a high-precision optical measurement instrument (not shown) or other high-precision distance measuring instruments (not shown) to ensure that the working distance is within the optimal range. Thereafter, the adjusted position (a position relative to the imaging lens 5) of the protective lens 33 is fastened by the working distance auxiliary adjustment system 4, ensuring that the adjusted working distance is not changed during subsequent use and preventing changes due to the external factor. After fastening completion, multiple times of verification inspection can be performed to ensure that under different object distance conditions, the self-adaptive endoscope working distance adjustment system 100 can still provide a clear and stable imaging effect.

In some examples, as shown in FIG. 1, the working distance auxiliary adjustment system 4 is configured as a first retaining ring 41 sleeved on the outer side of the support portion 21. At least part of an outer periphery of the support portion 21 forms a first guiding portion, and the sheath tube connection portion 32 is connected to the support portion 21 through the first guiding portion. When the sheath tube connection portion 32 is rotated axially relative to the support portion 21, the first guiding portion guides the sheath tube connection portion 32 to move axially, so that after an axial position of the protective lens 33 relative to the imaging lens 5 is adjusted, the first retaining ring 41 is axially abutted between the sheath tube connection portion 32 and the support portion 21. An axial thickness of the first retaining ring 41 is adjusted, so that the axial distance between the protective lens 33 and the imaging lens 5 can be precisely adjusted, thereby achieving the optimal object distance.

During adjustment, the sheath tube connection portion 32 is first rotated to fasten the protective sheath 3 onto the support portion 21. Then, the protective lens 33 is pressed against a surface of a living specimen (such as a pig liver, a pig kidney, and the like) to test the imaging effect. The sheath tube connection portion 32 is rotated, so that the first guiding portion guides the sheath tube connection portion 32 to move axially, thereby synchronously driving the protective lens 33 inside the outer sheath tube 31 to move axially. When a high-definition microscopic image is observed, the rotation of the sheath tube connection portion 32 is stopped to maintain an optimal imaging state. Subsequently, a distance D1 (as shown in FIG. 1) between an end of the sheath tube connection portion 32 and the lens holder 2 is measured by using a high-precision optical measuring instrument or other high-precision distance measuring instruments. According to a measurement result, the first retaining ring 41 of a proper thickness is selected. The protective sheath 3 is removed from the lens holder 2, the selected first retaining ring 41 of a proper thickness is sleeved on the outer side of the support portion 21 and abutted against the lens holder 2, and then the protective sheath 3 is reinstalled onto the support portion 21, so that the sheath tube connection portion 32 abuts against the first retaining ring 41, enabling an optimal working distance between the imaging lens 5 and the protective lens 33, thereby achieving the optimal object distance and ensuring high-quality microscopic imaging. Finally, the imaging effect is checked. If an image is blurred or unclear, a first retaining ring 41 of a similar size is selected for re-adjustment until the imaging achieves the optimal effect.

In some examples, the working distance auxiliary adjustment system 4 indirectly adjusts the axial distance between the protective lens 33 and the imaging lens 5 by using the thickness of the first retaining ring 41, thereby precisely controlling the working distance between the protective lens 33 and the imaging lens 5, and preventing changes in the working distance caused by an external factor or improper operation, so that the working distance between the protective lens 33 and the imaging lens 5 is maintained at a stable value.

In some examples, as shown in FIG. 2, the working distance auxiliary adjustment system 4 is configured as a second retaining ring 42 sleeved on the outer side of the image relay sheath 1. At least part of an outer periphery of the support portion 21 forms a first guiding portion, and the sheath tube connection portion 32 is connected to the support portion 21 through the first guiding portion. When the sheath tube connection portion 32 is rotated axially relative to the support portion 21, the first guiding portion guides the sheath tube connection portion 32 to move axially, so that after the axial position of the protective lens 33 relative to the imaging lens 5 is adjusted, the second retaining ring 42 is axially abutted between the sheath tube connection portion 32 and the support portion 21. An axial thickness of the second retaining ring 42 is adjusted, so that the axial distance between the protective lens 33 and the imaging lens 5 can be precisely adjusted, thereby achieving the optimal object distance.

During adjustment, the sheath tube connection portion 32 is first rotated to fasten the protective sheath 3 onto the support portion 21. Then, the protective lens 33 is pressed against a surface of a living specimen (such as a pig liver, a pig kidney, and the like) to test the imaging effect. The sheath tube connection portion 32 is rotated, so that the first guiding portion guides the sheath tube connection portion 32 to move axially, thereby synchronously driving the protective lens 33 inside the outer sheath tube 31 to move axially. When a high-definition microscopic image is observed, the rotation of the sheath tube connection portion 32 is stopped to maintain an optimal imaging state. Subsequently, a distance D2 between the sheath tube connection portion 32 and an end of the support portion 21 facing the object side is measured by using a high-precision optical measuring instrument or other high-precision distance measuring instruments (as shown in FIG. 2). According to the measurement result, a second retaining ring 42 of a proper thickness is selected. The protective sheath 3 is removed from the lens holder 2, the selected second retaining ring 42 of a proper thickness is sleeved on the outer side of the image relay sheath 1 and abutted against the end of the support portion 21 facing the object side, and then the protective sheath 3 is reinstalled onto the support portion 21, so that the sheath tube connection portion 32 abuts against the second retaining ring 42, enabling the optimal working distance between the imaging lens 5 and the protective lens 33, thereby achieving the optimal object distance and ensuring high-quality microscopic imaging. Finally, the imaging effect is inspected. If an image is blurred or unclear, a second retaining ring 42 of a similar size is selected for re-adjustment until the optimal imaging effect is achieved.

In some examples, the working distance auxiliary adjustment system 4 indirectly adjusts the axial distance between the protective lens 33 and the imaging lens 5 by using the thickness of the second retaining ring 42, thereby precisely controlling the working distance between the protective lens 33 and the imaging lens 5, and preventing changes in the working distance caused by an external factor or improper operation, so that the working distance between the protective lens 33 and the imaging lens 5 is maintained at a stable value.

In some examples, as shown in FIG. 1 and FIG. 2, the first guiding portion is configured as an external thread portion, and an internal thread portion engaged with the external thread portion is formed on an inner periphery of the sheath tube connection portion 32. Through engagement of the external thread portion (not shown) and the internal thread portion (not shown), the sheath tube connection portion 32 can be precisely rotated axially on the support portion 21, to precisely control an axial displacement distance of the protective lens 33 driven by the sheath tube connection portion 32, thereby precisely adjusting the working distance between the imaging lens 5 and the protective lens 33. After adjustment completion, the sheath tube connection portion 32 can be tightened through the engagement of the external thread portion and the internal thread portion, so that the sheath tube connection portion 32 is fastened on the support portion 21, preventing changes in the working distance between the imaging lens 5 and the protective lens 33 due to an external force or vibration during subsequent use.

In some examples, as shown in FIG. 1 and FIG. 2, the sheath tube connection portion 32 includes: a holding portion 321 for fastening the outer sheath tube 31, and an adjusting portion 322 forming the internal thread portion for connecting to the support portion 21. The holding portion 321 is configured to fasten the outer sheath tube 31 in the sheath tube connection portion 32, ensuring the stability of the outer sheath tube 31 and preventing loosening or shifting of the outer sheath tube 31 during use. The outer sheath tube 31 is securely fastened, so that other optical components (such as the imaging lens 5 and the protective lens 33) are indirectly protected, preventing external factors from affecting the optical components and ensuring the stability and clarity of imaging. The adjusting portion 322 is engaged with the external thread portion of the support portion 21 through the internal thread portion, so that the sheath tube connection portion 32 can move axially relative to the support portion 21, thereby adjusting the working distance between the imaging lens 5 and the protective lens 33. It can also be ensured that after adjustment, the sheath tube connection portion 32 and the support portion 21 are tightly connected, and a position of the adjusted protective lens 33 is fastened, so that the working distance between the adjusted imaging lens 5 and the protective lens 33 is maintained at a stable value, preventing changes due to external factors.

In some examples, as shown in FIG. 1, the lens holder 2 radially protrudes to form a protruding portion 23 that axially abuts against the first retaining ring 41, and the adjusting portion 322 axially abuts against a side of the first retaining ring 41 opposite to the protruding portion 23. The protruding portion 23 provides stable axial support for the first retaining ring 41, so that the first retaining ring 41 can be fastened and withstand an axial pressure formed by the abutment of the adjusting portion 322 during adjustment, ensuring that the first retaining ring 41 does not shift or loosen during working distance adjustment. The adjusting portion 322 axially abuts against a back side (namely, a side opposite to the protruding portion 23) of the first retaining ring 41, so that the first retaining ring 41 is ultimately fastened to the support portion 21, ensuring that the first retaining ring 41 can stably maintain at an original position after the working distance adjustment is completed, preventing accidental movement under an external force, and ensuring that the working distance between the imaging lens 5 and the protective lens 33 is maintained at a stable value, thereby ensuring the accuracy and long-term stability of the working distance adjustment.

In some examples, as shown in FIG. 2, the support portion 21 axially abuts against the second retaining ring 42, and the holding portion 321 axially abuts against a side of the second retaining ring 42 opposite to the support portion 21. The holding portion 321 axially abuts against a back side (namely, the side opposite to the support portion 21) of the second retaining ring 42 and cooperates with the support portion 21 to stably fasten an axial position of the second retaining ring 42, ensuring that a position of the second retaining ring 42 does not become loose after adjustment completion, and preventing the second retaining ring 42 from shifting due to an external factor, thereby preventing deviation of the working distance. In addition, under the combined abutment of the support portion 21 and the holding portion 321, the second retaining ring 42 can ensure the adjustment accuracy of the working distance between the imaging lens 5 and the protective lens 33, and after adjustment completion, stably maintain the working distance between the imaging lens 5 and the protective lens 33, preventing changes in the working distance due to an external factor, thereby ensuring the quality of microscopic imaging.

In some examples, as shown in FIG. 3, the support portion 21 is provided with a limiting portion 211 to restrict axial rotation of the sheath tube connection portion 32. The working distance auxiliary adjustment system 4 is configured as a movable cylinder 43 partially sleeved on the outer side of the sheath tube connection portion 32 and rotatably connected to the lens holder 2. An inner periphery of the movable cylinder 43 is provided with a second guiding portion. When the movable cylinder 43 is rotated relative to the sheath tube connection portion 32, the second guiding portion guides the sheath tube connection portion 32 to move axially along the support portion 21, to adjust an axial position of the protective lens 33 relative to the imaging lens 5. During adjustment, the protective lens 33 is pressed against a surface of a living specimen (such as a pig liver, a pig kidney, and the like) to test the imaging effect. The movable cylinder 43 is rotated, so that the second guiding portion guides the sheath tube connection portion 32 to move along the support portion 21, thereby synchronously driving the protective lens 33 inside the outer sheath tube 31 to move axially. When a high-definition microscopic image is observed, the rotation of the movable cylinder 43 is stopped to maintain the optimal imaging state, so that the working distance between the imaging lens 5 and the protective lens 33 no longer changes, and the optimal working distance is provided between the imaging lens 5 and the protective lens 33, thereby achieving the optimal object distance and ensuring high-quality microscopic imaging. During the rotation of the movable cylinder 43, the second guiding portion can guide the sheath tube connection portion 32 to move smoothly along the support portion 21, ensuring the smoothness and accuracy of axial movement of the protective lens 33 relative to the imaging lens 5, thereby precisely adjusting the working distance between the protective lens 33 and the imaging lens 5.

The working distance auxiliary adjustment system 4 actively adjusts the axial distance of the protective lens 33 relative to the imaging lens 5 by rotating the movable cylinder 43, thereby precisely controlling the working distance between the protective lens 33 and the imaging lens 5 and maintaining the working distance between the protective lens 33 and the imaging lens 5 at a stable value.

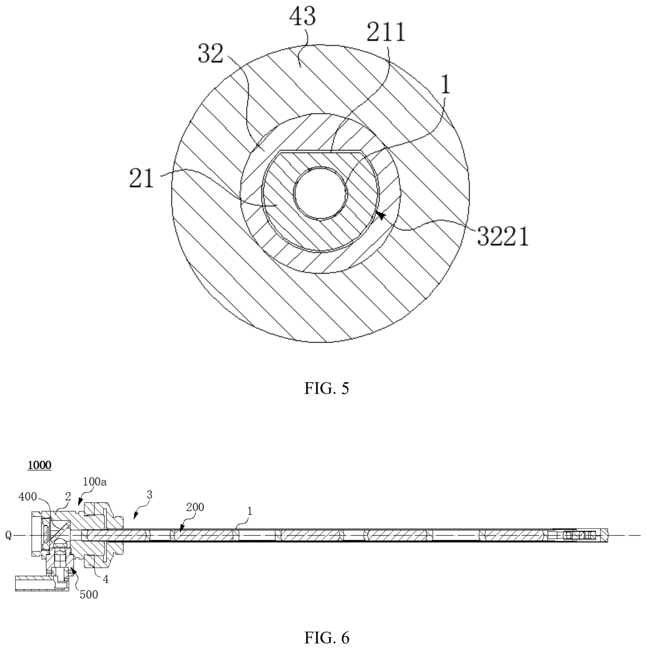

In some examples, the limiting portion 211 can be configured as a limiting surface formed by a recess on the outer periphery of the support portion 21 as shown in FIG. 5. The limiting surface prevents the sheath tube connection portion 32 from rotating, thereby ensuring that the protective lens 33 inside the outer sheath tube 31 is precisely moved along a predetermined axial path during adjustment and ensuring the stability of a final working distance. The limiting portion 211 can also be configured as other structures capable of restricting the rotation of the sheath tube connection portion 32, which is not limited in the present disclosure.

In some examples, as shown in FIG. 3, the second guiding portion is configured as an internal thread portion, and the outer periphery of the sheath tube connection portion 32 is provided with an external thread portion engaged with the internal thread portion. Through engagement of the internal thread portion (not shown) and the external thread portion (not shown), an axial displacement distance of the protective lens 33 relative to the imaging lens 5 can be precisely adjusted. The sheath tube connection portion 32 is rotated, so that the axial displacement distance of the protective lens 33 relative to the imaging lens 5 can be gradually and precisely adjusted, ensuring that the working distance between the imaging lens 5 and the protective lens 33 is within the optimal range, thereby achieving the best imaging effect. After the adjustment of the axial displacement distance of the protective lens 33 relative to the imaging lens 5 is completed, an adjusted position of the protective lens 33 is fastened through the engagement of the internal thread portion (not shown) and the external thread portion (not shown), to stably fasten the working distance between the adjusted protective lens 33 and the imaging lens 5, preventing the position of the protective lens 33 from changing due to an external factor during subsequent use.

In some examples, as shown in FIG. 3, the working distance auxiliary adjustment system 4 further includes: a bearing 44 disposed between the lens holder 2 and the movable cylinder 43, where the movable cylinder 43 is axially rotatable relative to the lens holder 2 through the bearing 44. The bearing 44 is disposed between the lens holder 2 and the movable cylinder 43, so that the movable cylinder 43 rotates axially smoothly during working distance adjustment, reducing a friction and resistance during the rotation of the movable cylinder 43, making the rotation of the movable cylinder 43 smoother and more stable, preventing resistance or jamming, thereby improving the efficiency of the working distance adjustment process and ensuring accuracy during adjustment.

In some examples, as shown in FIG. 3, the movable cylinder 43 includes: a fastening cylinder section 431 securely connected to the bearing 44, and an adjusting cylinder section 432 forming an internal thread portion and sleeved on the outer side of the sheath tube connection portion 32. The bearing 44 is connected to the fastening cylinder section 431 to provide stable rotational support for the movable cylinder 43 and maintain smoothness during adjustment. The adjusting cylinder section 432 is engaged with the external thread portion of the sheath tube connection portion 32 through the internal thread portion of the adjusting cylinder section 432. The adjusting cylinder section 432 is rotated, to adjust the working distance between the protective lens 33 and the imaging lens 5.

In some examples, refer to FIG. 3 and FIG. 5. The sheath tube connection portion 32 includes: a holding portion 321 for fastening the outer sheath tube 31, and an adjusting portion 322 forming the external thread portion. The adjusting portion 322 is provided with a movable groove 3221 that matches an external contour of the support portion 21. The holding portion 321 is configured to fasten the outer sheath tube 31 in the sheath tube connection portion 32, ensuring the stability of the outer sheath tube 31 and preventing loosening or shifting of the outer sheath tube 31 during use. The movable groove 3221 matches the external contour of the support portion 21, so that the adjusting portion 322 can move axially along the support portion 21, preventing rotation or radial displacement, thereby ensuring the accuracy and stability of the protective lens 33 during adjustment.

In some examples, refer to FIG. 3. The working distance auxiliary adjustment system 4 further includes: a locking member 45 that radially penetrates a wall of the adjusting cylinder section 432 to abut against the sheath tube connection portion 32. The locking member 45 abuts against the sheath tube connection portion 32 by radially penetrating the wall of the adjusting cylinder section 432, thereby securely fastening an adjusted position of the protective lens 33, preventing accidental displacement or loosening of a relative position between the adjusting cylinder section 432 and the sheath tube connection portion 32 after adjustment completion, and ensuring that, during use, the working distance between the protective lens 33 and the imaging lens 5 does not shift due to external vibration, impact, or operation. Further, positions of the adjusting cylinder section 432 and the sheath tube connection portion 32 are fastened by using the locking member 45, so that long-term stability of the optimal working distance between the imaging lens 5 and the protective lens 33 is further ensured.

In some examples, refer to FIG. 4. The working distance auxiliary adjustment system 4 is configured as a spacer ring 46 axially abutted between the imaging lens 5 and the protective lens 33, to adjust an axial position of the protective lens 33 relative to the imaging lens 5. The axial thickness of the spacer ring 46 is adjusted, to precisely adjust the distance between the protective lens 33 and the imaging lens 5, thereby achieving the optimal object distance. During adjustment, after the imaging lens 5 is installed into the image relay sheath 1, the working distance of the imaging lens 5 is optically inspected, in particular to determine an optimal working distance of the imaging lens 5. After the optimal working distance is determined, a difference between the protective lens 33 and the optimal working distance is measured by using a high-precision optical measuring instrument or other high-precision distance measuring instruments. In particular, after the optimal working distance of the imaging lens 5 is determined, a working distance between the protective lens 33 and the imaging lens 5 is measured by using a high-precision optical measuring instrument or other high-precision distance measuring instruments. Then, a difference between the working distance between the protective lens 33 and the imaging lens 5 and the previously described optimal working distance is measured by using the high-precision optical measuring instrument or other high-precision distance measuring instruments. A spacer ring 46 of a proper thickness is selected based on a measurement result, the selected spacer ring 46 of a proper thickness is installed inside the outer sheath tube 31, and then the protective sheath 3 is installed onto the support portion 21, so that the spacer ring 46 is axially abutted between the protective lens 33 and the imaging lens 5, enabling the optimal working distance between the imaging lens 5 and the protective lens 33, thereby achieving the optimal object distance and ensuring high-quality microscopic imaging. Finally, the imaging effect is checked. If an image is blurred or unclear, a spacer ring 46 of a similar size is selected for re-adjustment until the optimal imaging effect is achieved.

In some examples, the working distance auxiliary adjustment system 4 indirectly adjusts the axial distance between the protective lens 33 and the imaging lens 5 by using a thickness of the spacer ring 46, thereby precisely controlling the working distance between the protective lens 33 and the imaging lens 5 and preventing changes in the working distance caused by an external factor or improper operation, so that the working distance between the protective lens 33 and the imaging lens 5 is maintained at a stable value.

In some examples, after the protective sheath 3 is sleeved onto the support portion 21, a sealed space is formed between the protective sheath 3 and the image relay sheath 1. After the adjustment of the distance between the protective lens 33 and the imaging lens 5 is completed, the sealed space is formed between the protective sheath 3 and the image relay sheath 1, thereby isolating contaminants such as dust and moisture from an external environment from affecting the imaging lens 5 and the protective lens 33, and maintaining the quality of microscopic imaging. Especially in the medical field, the sealed space can ensure that the self-adaptive endoscope working distance adjustment system 100 maintains a sterile state in sensitive environments such as surgery, reducing a risk of external microorganisms or pathogens entering the self-adaptive endoscope working distance adjustment system 100, thereby ensuring the hygiene and safety of the self-adaptive endoscope working distance adjustment system 100. In addition, the sealed space can better protect an optical path and imaging effect of the self-adaptive endoscope working distance adjustment system 100, reducing the impact of environmental factors (such as temperature and humidity changes) on the system, thereby improving the quality of microscopic imaging and the reliability of the system.

It should be noted that spacings between the first retaining ring 41, the second retaining ring 42, and the spacer ring 46 of different thicknesses can be accurate to 0.01 mm. During a process in which the movable cylinder 43 guides, through the second guiding portion, the sheath tube connection portion 32 to move axially along the support portion 21, the accuracy of axial displacement of the protective lens 33 relative to the imaging lens 5 can also reach 0.01 mm, to ensure precise adjustment of the final working distance. For example, during adjustment, after the optimal working distance has been determined and preliminarily measured, and the first retaining ring 41 (or second retaining ring 42, spacer ring 46) of a proper thickness has been selected and installed in the self-adaptive endoscope working distance adjustment system 100, the working distance has already been close to an ideal value. However, under high-precision measurement, if there is still an error of 0.01 mm, a new first retaining ring 41 with a thickness of ±0.01 mm relative to the original first retaining ring 41 may be selected and reinstalled in the self-adaptive endoscope working distance adjustment system 100, to finely adjust the working distance and achieve the optimal object distance.

Based on any of the technical solutions of the self-adaptive endoscope working distance adjustment system 100 as disclosed in the above embodiments and proper combinations, this embodiment also discloses an endoscopic imaging system 1000.

Refer to FIG. 6. The endoscopic imaging system 1000 includes: the self-adaptive endoscope working distance adjustment system 100 as disclosed in the above embodiments, a relay lens 200 coaxially disposed within an image relay sheath 1 included in the self-adaptive endoscope working distance adjustment system 100, a dichroic mirror 400 disposed within a lens holder 2 included in the self-adaptive endoscope working distance adjustment system 100, and a light source 500.

The lens holder 2 receives incident light (not shown) from the light source 500 onto the dichroic mirror 400, the incident light is reflected by the dichroic mirror 400 and sequentially enters the relay lens 200 and an imaging lens 5. The imaging lens 5 then focuses the light onto an object under inspection W, a tissue surface of the object under inspection W reflects the light, and the imaging lens 5 collects and images the reflected light from the object under inspection W. The relay lens 200 then transmits an image formed by the imaging lens 5 at a 1:1 ratio. The reflected light passes through the dichroic mirror 400 and is captured by the endoscopic imaging system 1000, thereby allowing operators (for example, medical personnel or researchers) to observe images of biological tissue.

Based on the endoscopic imaging system 1000 as disclosed in the above embodiments, this embodiment also discloses an application of the endoscopic imaging system, in which the endoscopic imaging system 1000 as disclosed in the above embodiment is configured to perform optical imaging on biological tissue (including living organisms or excised biological tissues), thereby allowing operators (for example, medical personnel or researchers) to observe images of the biological tissue. Refer to the above description for details, which are not described herein again.

The series of detailed descriptions listed in the preceding text are only specific explanations for the feasible implementations of the present invention. They are not intended to limit the protection scope of the present invention.

In particular, the endoscope of any of the above described aspects and embodiments is a endomicroscope.

For those skilled in the art, it is apparent that the present invention is not limited to the details of the exemplary embodiments mentioned above. Moreover, the present invention can be implemented in other specific forms without departing from the scope of the invention as defined by the appended claims. Therefore, from any perspective, the embodiments should be regarded as exemplary and non-limiting. The scope of the present invention is defined by the appended claims rather than the above description. Any reference signs in the claims should not be construed as limitation on the related claims.

Additionally, it should be understood that though this specification is described according to the implementations, not every implementation merely includes a single independent technical solution. This manner of description in the specification is solely for clarity. Those skilled in the art should consider the specification as a whole, and the technical solutions in various embodiments can also be appropriately combined to form other implementations understandable by those skilled in the art.

## Claims

1. A self-adaptive endoscope working distance adjustment system (100), **characterized by** comprising:
an image relay sheath (1), a lens holder (2) disposed on an image side of the image relay sheath (1), a protective sheath (3) sleeved on an outer side of the image relay sheath (1), and a working distance auxiliary adjustment system (4) disposed between the protective sheath (3) and the lens holder (2) and/or between the protective sheath (3) and the image relay sheath (1), **characterized in that**
the lens holder (2) axially protrudes to form a support portion (21), and an imaging lens (5) is disposed inside the image relay sheath (1) away from the image side;
the protective sheath (3) comprises: an outer sheath tube (31) axially extending over the imaging lens (5), a sheath tube connection portion (32) partially sleeved on an outer side of the support portion (21) and connected to the outer sheath tube (31), and a protective lens (33) disposed at an object-side end inside the outer sheath tube (31); and
the working distance auxiliary adjustment system (4) is configured to adjust a relative axial position between the imaging lens (5) and the protective lens (33) and to maintain a working distance between the protective lens (33) and the imaging lens (5).

2. The self-adaptive endoscope working distance adjustment system (100) according to claim 1, **characterized in that** the working distance auxiliary adjustment system (4) is configured as a first retaining ring (41) sleeved on the outer side of the support portion (21);
at least part of an outer periphery of the support portion (21) forms a first guiding portion, and the sheath tube connection portion (32) is connected to the support portion (21) through the first guiding portion; and
when the sheath tube connection portion (32) is rotated axially relative to the support portion (21), the first guiding portion guides the sheath tube connection portion (32) to move axially, so that after an axial position of the protective lens (33) relative to the imaging lens (5) is adjusted, the first retaining ring (41) is axially abutted between the sheath tube connection portion (32) and the support portion (21).

3. The self-adaptive endoscope working distance adjustment system (100) according to claim 1, **characterized in that** the working distance auxiliary adjustment system (4) is configured as a second retaining ring (42) sleeved on the outer side of the image relay sheath (1);
at least part of an outer periphery of the support portion (21) forms a first guiding portion, and the sheath tube connection portion (32) is connected to the support portion (21) through the first guiding portion; and
when the sheath tube connection portion (32) is rotated axially relative to the support portion (21), the first guiding portion guides the sheath tube connection portion (32) to move axially, so that after an axial position of the protective lens (33) relative to the imaging lens (5) is adjusted, the second retaining ring (42) is axially abutted between the sheath tube connection portion (32) and the support portion (21).

4. The self-adaptive endoscope working distance adjustment system (100) according to claim 2, **characterized in that** after the protective sheath (3) is sleeved onto the support portion (21), a sealed space is formed between the protective sheath (3) and the image relay sheath (1).

5. The self-adaptive endoscope working distance adjustment system (100) according to claim 3, **characterized in that** after the protective sheath (3) is sleeved onto the support portion (21), a sealed space is formed between the protective sheath (3) and the image relay sheath (1).

6. The self-adaptive endoscope working distance adjustment system (100) according to claim 2, **characterized in that** the first guiding portion is configured as an external thread portion, and an internal thread portion engaged with the external thread portion is formed on an inner periphery of the sheath tube connection portion (32).

7. The self-adaptive endoscope working distance adjustment system (100) according to claim 3, **characterized in that** the first guiding portion is configured as an external thread portion, and an internal thread portion engaged with the external thread portion is formed on an inner periphery of the sheath tube connection portion (32).

8. The self-adaptive endoscope working distance adjustment system (100) according to claim 6, **characterized in that** the sheath tube connection portion (32) comprises: a holding portion (321) for fastening the outer sheath tube (31), and an adjusting portion (322) forming the internal thread portion for connecting to the support portion (21).

9. The self-adaptive endoscope working distance adjustment system (100) according to claim 7, **characterized in that** the sheath tube connection portion (32) comprises: a holding portion (321) for fastening the outer sheath tube (31), and an adjusting portion (322) forming the internal thread portion for connecting to the support portion (21).

10. The self-adaptive endoscope working distance adjustment system (100) according to claim 8, **characterized in that** the lens holder (2) radially protrudes to form a protruding portion (23) that axially abuts against the first retaining ring (41), and the adjusting portion (322) axially abuts against a side of the first retaining ring (41) opposite to the protruding portion (23).

11. The self-adaptive endoscope working distance adjustment system (100) according to claim 9, **characterized in that** the support portion (21) axially abuts against the second retaining ring (42), and the holding portion (321) axially abuts against a side of the second retaining ring (42) opposite to the support portion (21).

12. An endoscopic imaging system (1000), **characterized by** comprising: the self-adaptive endoscope working distance adjustment system (100) according to claim 2, a relay lens (200) coaxially disposed within an image relay sheath (1) comprised in the self-adaptive endoscope working distance adjustment system (100), a dichroic mirror (400) disposed within a lens holder (2) comprised in the self-adaptive endoscope working distance adjustment system (100), and a light source (500); and
the endoscopic imaging system (1000) is configured to perform optical imaging on biological tissue.

13. An endoscopic imaging system (1000), **characterized by** comprising: the self-adaptive endoscope working distance adjustment system (100) according to claim 3, a relay lens (200) coaxially disposed within an image relay sheath (1) comprised in the self-adaptive endoscope working distance adjustment system (100), a dichroic mirror (400) disposed within a lens holder (2) comprised in the self-adaptive endoscope working distance adjustment system (100), and a light source (500); and
the endoscopic imaging system (1000) is configured to perform optical imaging on biological tissue.
